(12) **EUROPÄISCHE PATENTSCHRIFT**

(45) Veröffentlichungstag und Bekanntmachung des Hinweises auf die Patenterteilung:
**24.04.1996 Patentblatt 1996/17**

(21) Anmeldenummer: **92919355.5**

(22) Anmeldetag: **12.09.1992**

(51) Int. Cl.$^6$: **A61B 5/0275**

(86) Internationale Anmeldenummer: **PCT/DE92/00796**

(87) Internationale Veröffentlichungsnummer:
**WO 93/05704 (01.04.1993 Gazette 1993/09)**

(54) **VERFAHREN UND VORRICHTUNG ZUR ERMITTLUNG DES ZIRKULIERENDEN BLUTVOLUMENS**

PROCESS AND DEVICE FOR DETERMINING THE VOLUME OF BLOOD IN CIRCULATION

PROCEDE ET DISPOSITIF POUR DETERMINER LE VOLUME DE SANG EN CIRCULATION

(84) Benannte Vertragsstaaten:
**AT BE CH DE FR GB LI**

(30) Priorität: **13.09.1991 DE 4130931**

(43) Veröffentlichungstag der Anmeldung:
**01.09.1993 Patentblatt 1993/35**

(73) Patentinhaber: **Hoeft, Andreas, Dr. med.**
**D-37073 Göttingen (DE)**

(72) Erfinder: **Hoeft, Andreas, Dr. med.**
**D-37073 Göttingen (DE)**

(74) Vertreter: **Patentanwälte Thömen & Körner**
**Zeppelinstrasse 5**
**D-30175 Hannover (DE)**

(56) Entgegenhaltungen:
**EP-A- 0 359 206**

- **BIOMEDIZINISCHE TECHNIK Bd. 28, Nr. 10, Oktober 1983, BERLIN (DE) Seiten 216 - 220 HOEFT ET AL. 'Messung der Koronardurchblutung mit einem Doppelfiberoptiksystem und rechnergestuetzter Entfaltung von transkoronaren Farbstoffdilutionssignalen'**
- **TOHOKU J. EXP. MED. Nr. 148, 1986, Seiten 49 - 56 K. HANEDA ET AL 'A Method for Measurement of Total Circulating Blood Volume Using Indocyanine Green''**
- **COMPUTER PROGRAMS IN BIOMEDICINE Nr. 11, 1980, AMSTERDAM (NL) Seiten 105 - 112 M. A. COLE ET AL. 'Rapif Assessment of Cardiac Output and Central Blood Volumes from Indicator Dilution Curves'**

**Beschreibung**

Die Erfindung betrifft ein Verfahren zur Ermittlung des zirkulierenden Blutvolumens nach dem Oberbegriff des Anspruchs 1.

Eine normale Funktion des Herz-Kreislauf Systems ist entscheidend von der Füllung der Blutgefäße mit einem adäquaten Blutvolumen abhängig. Ein Mangel an Blut kann ebenso wie ein Überfüllung zu einem lebensbedrohlichen Kreislaufversagen führen. Im Rahmen der Intensivmedizin sowie der perioperativen anaesthesiologischen Therapie besteht daher ein wesentliches Ziel darin, trotz zum Teil großer Blut- und/oder Flüssigkeitsverluste einen adäquaten Füllungszustand des Kreislaufsystems zu gewährleisten. Hierzu stützt sich die therapeutische Entscheidungsfindung vornehmlich auf indirekte Größen wie Blutdruck, Herzfrequenz und zentralen Venendruck, da zur Zeit direkte Messungen des Blutvolumens technisch aufwendig und unter klinischen Bedingungen kaum durchführbar sind.

Ein weiteres Anwendungsgebiet für die Messung des Blutvolumens ist der Leistungssport, bei dem die Kenntnis der Einflüsse von Trainingsmethoden, Kleidung und Ernährung auf das zirkulierende Blutvolumen zur Optimierung der Leistung und Ausdauer der Athleten ausgewertet wird. Dabei kommen nur solche Meßverfahren in Betracht, die nur eine kurze Meßzeit benötigen und das Meßergebnis nicht durch die Meßmethode selbst beeinflussen.

Die nuklearmedizinischen Verfahren, die zur Verfügung stehen, sind aufgrund der Strahlenbelastung für den Patienten bzw. den Leistungssportler und das Personal sowie aufgrund der aufwendigen Technik nicht im intensivmedizinischen und perioperativen Bereich sowie ebenfalls nicht beim Leistungssport einsetzbar.

Alternativ kann das Blutvolumen auch mit Hilfe von Indikatorverdünnungsmethoden und Farbstoffen als Indikator bestimmt werden. Hierbei wird meistens Indocyaningrün (ICG) verwendet, das nach Injektion in den Kreislauf vollständig an Albumin, also an Bluteiweiß gebunden wird und daher das Gefäßsystem nicht verläßt. Die Meßmethodik beruht auf dem Prinzip der Massenerhaltung, d.h., daß nach Injektion einer bekannten Menge des ICG und Verteilung im gesamten Blutvolumen aus der resultierenden Konzentration auf das Verteilungsvolumen für den Indikator, das dem Blutvolumen entspricht, geschlossen werden kann.

Die Konzentration ist jedoch nicht direkt meßbar, da der Farbstoff bereits durch die Leber ausgeschieden wird, bevor es zu einer homogenen Verteilung im Kreislauf gekommen ist. Die Halbwertszeit des ICG liegt bei einer normalen Leberfunktion zwischen 3 und 5 Minuten. Man muß daher, um auf die im Zeitpunkt t = 0 vorliegende virtuelle Konzentration schließen zu können, mehrere Blutproben über einen Zeitraum von mindestens 15 Minuten nach der Injektion entnehmen, um aus dem Abfall der Konzentration die Eliminationskinetik des Farbstoffes durch die Leber zu erfassen und mittels Rückextrapolation, meist exponentieller Art, auf die ideale Konzentration zum Zeitpunkt der Injektion zu schließen.

Auch dieses Verfahren ist klinisch nur bedingt einsetzbar, da sowohl ein großer Zeitaufwand für die Gewinnung und Verarbeitung der Blutproben notwendig ist, als auch eine nicht zu vernachlässigende Menge Blut dem Patienten entnommen werden muß.

Bei einer Weiterentwicklung des oben beschriebenen Verfahrens, wie es auch aus Tohoku J. Exp. Med. No. 148, 1986, Seiten 49 - 56, K. Haneda et al, "A Method Measurement of Total Circulating Blood Volume Using Indocyanine Green" bekannt ist, wurde daher versucht, den Farbstoffnachweis direkt im Gefäßsystem mit Hilfe von Fiberoptikkathetern durchzuführen. Nach einer Bolusinjektion von ICG mittels eines zentralen Venenkatheters in den rechten Vorhof und Registrierung des Konzentrationszeitverlaufes in der Aorta, also der Hauptschlagader erhält man einen typischen Verlauf einer Farbstoffverdünnungskurve. Nach einer ersten Passage des Farbstoffes vom Injektionsort durch den Lungenkreislauf ist ein Wiederanstieg der Konzentration zu beobachten, der aus der Rezirkulation des Indikators resultiert. Erst nach mehreren Kreislaufpassagen des Farbstoffes kann die Verteilungsphase als abgeschlossen betrachtet werden und erst danach könnte aus dem langsamen, kaum sichtbaren Abfall der Indikatorkonzentration die Eliminationskinetik des Farbstoffes durch die Leber ermittelt werden.

Das wesentliche Problem dieses Ansatzes besteht darin, daß einerseits eine genügend lange Zeit abgewartet werden muß, bevor die Verteilungsphase des Farbstoffes abgeschlossen ist, daß jedoch andererseits mit zunehmender Zeit die Farbstoffkonzentration immer geringer wird und daher häufig die Nachweisgrenze für die fiberoptische Farbstoffmessung unterschritten wird.

Aus Biomedizinische Technik, Vol. 28, No. 10 Oktober 1983, Berlin (DE), Seiten 216 -220, Hoeft et al, "Messung der Koronardurchblutung mit einem Doppelfiberoptiksystem und rechnergestützter Entfaltung von transkoronaren Farbstoffdilutionssignalen" ist außerdem bekannt, den arteriellen Konzentrationszeitverlauf und den venösen Konzentrationszeitverlauf an einem Herzen nach venöser Farbstoffinjektion zu messen. Bei Betrachtung des arteriellen Konzentrationszeitverlaufs als Eingangsfunktion und des venösen Konzentrationszeitverlaufs als Ausgangsfunktion erhält man nach Mittelung und Enfaltung der Eingangs- und Ausgangsfunktionen die Gewichtsfunktion und Übergangsfunktion, aus denen sich ein Fluß-Verteilungsvolumenverhältnis und ein Verteilungsvolumen errechnen läßt. Dieser Vorgehensweise liegt ein Teilkreislauf zugrunde, der als offenes System zu betrachten ist.

Schließlich ist aus Computer Programs in Biomedicine, No. 11, 1980, Amsterdam (NL), Seiten 105 - 112, M. A. Cole et al, "Rapid Assessment of Cardiac Output and Central Blood Volumes from Indicator Dilution Curves" bekannt, Die Herzleistung, das zentrale Blutvolumen und die Transitzeit zu messen indem der einmalige Anstieg und nachfolgende Abfall der Farbstoffkonzentration nach Injektion eines Farbstoffes in den Blut an einem Meßort bestimmt wird. Auch bei diesem Ansatz wird ein Teilkreislauf als offenes System betrachtet.

Der Erfindung liegt die Aufgabe zugrunde, ein Verfahren zur Ermittlung des zirkulierenden Blutvolumens zu entwickeln, bei der der gesamte Informationsgehalt der optisch gemessenen Farbstoffverdünnungskurve für die Berechnung der Eliminationskinetik ausgenutzt wird.

Diese Aufgabe wird bei einem Verfahren nach dem Oberbegriff des Anspruchs 1 durch die im Kennzeichen angegebenen Merkmale gelöst.

Die Berechnung des zirkulierenden Blutvolumens aus der Kreislauftransportfunktion beruht auf der Auswertung einer kompartiellen Analyse der Farbstoffverdünnungskurve. Ein wesentlicher Vorteil gegenüber dem eingangs erwähnten in-vitro Verfahren besteht darin, daß das erfindungsgemäße Verfahren keine Blutentnahmen benötigt, die vor allem bei Mehrfachbestimmungen beim Patienten zu nicht unerheblichen Blutverlusten führen können. Ebenso ist der reduzierte Zeitbedarf hervorzuheben. Als Meßzeitdauer nach Farbstoffinjektion scheint nach den bisherigen Erfahrungen ein Zeitraum von 3 - 4 Minuten ausreichend zu sein.

Die in-vitro Bestimmung erfordert hingegen Blutprobenentnahmen über einen Zeitraum von mindestens 15 Minuten. Des weiteren müssen hierbei die Blutproben relativ aufwendig spektralphotometrisch gemessen und ausgewertet werden. Das Ergebnis der Messung dürfte in aller Regel bei dem in-vitro Verfahren nicht vor einer Stunde nach Messung zur Verfügung stehen.

Der Vorteil des erfindungsgemäßen Verfahrens gegenüber dem ebenfalls erwähnten fiberoptischen Farbstoffnachweis besteht darin, daß für die Berechnung der Parameter der Kreislauftransportfunktion der Informationsgehalt der gesamten Farbstoffverdünnungskurve ausgenutzt wird. Die Farbstoffelimination, die auch während der Verteilungsphase stattfindet, wird somit bei dieser Lösung mit erfaßt.

Bei den bisherigen Ansätzen zur Bestimmung des zirkulierenden Blutvolumens mittels fiberoptischem Farbstoffnachweis wird versucht, nach Abklingen der Verteilungsphase des Farbstoffes eine monoexponentielle Extrapolation des Farbstoffsignals durchzuführen. Hierbei ist ein wesentlicher Nachteil, daß die Farbstoffkonzentrationen zu diesem Zeitpunkt sehr niedrig sind und zum Teil unterhalb der Nachweisgrenze liegen können. Die Extrapolation im späten Bereich der Farbstoffverdünnungskurve ist daher mit großen Unsicherheiten behaftet. Eine Validierung dieses Verfahrens liegt bislang auch noch nicht vor.

Für die Berechnung des Blutvolumens aus der Kreislauftransportfunktion bieten sich zwei Alternativen. Einmal kann die Kreislaufimpulsantwort bestimmt werden, die einer idealen Indikatorinjektion in extrem kurzer Zeit zu einem Zeitpunkt t = 0 entsprechen würde. Aus der Kreislaufimpulsantwort wird durch Rückextrapolation oder direkte Berechnung aus den Parametern der Kreislauftransportfunktion die virtuelle Farbstoffkonzentration zum Zeitpunkt t = 0 bestimmt. Das zirkulierende Blutvolumen berechnet sich dann aus der vorgegebenen Menge des injizierten Indikatorfarbstoffes und der virtuellen Farbstoffkonzentration zum Zeitpunkt t = 0.

Zum anderen kann die mittlere Transitzeit $m_{tt}$ bestimmt wird, die der Indikatorfarbstoff benötigt, um den Kreislauf oder einen Teilkreislauf zu passieren. Das Blutvolumen berechnet sich aus dem Volumenstrom und der mittleren Transitzeit.

In der praktischen Durchführung des Verfahrens wird die erste Alternative bevorzugt. Auch hier besteht ein wesentlicher Vorteil gegenüber den eingangs genannten Verfahren. Die Problematik bei der konventionellen monoexponentiellen Rückextrapolationstechnik liegt nämlich in der Definition des Injektionszeitpunktes, d.h. in der Wahl einer korrekten Zeitachse, von der entscheidend die Berechnung bei der Rückextrapolation abhängt.

Die Farbstoffinjektion kann jedoch nur in endlicher Zeit erfolgen, so daß hilfsweise der mittlere Injektionszeitpunkt als Bezugszeitpunkt definiert werden muß. Es ist jedoch zu bedenken, daß zu diesem Zeitpunkt der Indikator noch nicht das arterielle Gefäßsystem erreicht hat, und von daher die Elimination des Indikators noch nicht begonnen hat. Idealerweise wäre der Zeitpunkt mit demjenigen Zeitpunkt gleichzusetzen, der dem Beginn der Elimination entspricht.

Diese Problematik besteht bei der Berechnung des zirkulierenden Blutvolumens auf der Basis einer Kreislaufimpulsantwort nicht, da diese unabhängig von der Form und dem Zeitpunkt der ersten Passage ist. Es besteht daher auch Unabhängigkeit gegenüber dem Injektionszeitpunkt, da definitionsgemäß bei der Kreislaufimpulsantwort die Indikatoreinbringung in Form eines Dirac-Impulses zum Zeitpunkt t = 0 erfolgt.

Bei einer praktischen Anwendung des Verfahrens wird die Berechnung der Kreislauftransportfunktion (g(t)) aus der gemessenen Farbstoffkonzentration mit Hilfe eines iterativen, nichtlinearen Anpassungsverfahrens durchgeführt. Unter Vorgabe einer Modellfunktion

$$g(t) = a_m g_m(t) + a_{m+1} g_{m+1}(t) + \dots + a_n g_n(t)$$

$$\text{mit} \sum_{m=1}^{n} a_m = 1,$$

bei der die einzelnen Kompartimente $a_m g_m$ durch linksschiefe Verteilungsfunktionen beschrieben sind, wird wiederholt eine rekursive Faltung gemäß den Gleichungen

$$c(t) = c_{bolus}(t) + c_{rez}(t)$$

und

$$c_{rez}(t) = k * \int_0^t g(t-u) * c(u)\, du$$

durchgeführt und die Parameter k, $a_m$ sowie die Parameter der Verteilungsfunktionen werden nach dem Verfahren der kleinsten quadratischen Abweichung optimiert. Dabei wird wenigstens ein Kompartiment $a_1 g_1(t)$ angesetzt. Weiterhin bilden c(t) den Konzentrationszeitverlauf des Indikatorfarbstoffes, $c_{bolus}(t)$ den den Meßort direkt passierenden ersten Anteil des Farbstoffkonzentrationszeitverlaufs, $c_{rez}(t)$ einen rezirkulierender Anteil des Farbstoffkonzentrationszeitverlaufs und k die Eliminationsfraktion des durch die Leber eliminierten Farbstoffes.

Diesem Berechnungsverfahren liegt ein vereinfachtes Kreislaufmodell zugrunde, das jedoch die tatsächlichen Verhältnisse ausreichend genau nachbildet. Das Verfahren beruht auf den Prinzipien der stochastischen Systemanalyse, wobei aus der Rezirkulationskinetik des Farbstoffes eine Kreislauftransportfunktion für den Farbstoff berechnet wird.

Vorzugsweise werden bei der Berechnung der Kreislauftransportfunktion zwei Kompartimente $a_1 g_1(t)$ und $a_2 g_2(t)$ angesetzt.

Diese Auswahl beruht auf Voruntersuchungen, die bei Farbstoffverdünnungskurven an Patienten gemessen worden sind. Es handelt sich hierbei um ein schnelles und ein langsames Kompartiment.

Gemäß einer ersten Alternative kann die optische Messung der im Blutkreislauf resultierenden Farbstoffkonzentration durch fiberoptische Messung in einem zentralen Gefäß vorgenommen werden.

Diese Meßmethode zeichnet sich durch hohe Meßempfindlichkeit bei verhältnismäßig geringem Aufwand aus.

Eine andere Alternative sieht vor, daß die optische Messung der im Blutkreislauf resultierenden Farbstoffkonzentration nicht-invasiv mittels Licht-Transmissions- oder Reflexionsmessung eingestrahlten Lichtes an geeigneten Körperorten, insbesondere am Finger, Ohrläppchen, Nasenrücken, Wangenschleimhaut oder Stirn durchgeführt wird.

Der meßtechnische Aufwand ist hier höher, dafür ist die Meßmethode aber schonend für den Patienten und besonders geeignet für Messungen, die die Körperfunktionen nicht beeinträchtigen dürfen, was gerade im Leistungssport von Bedeutung ist.

Die Erfindung betrifft ferner eine Vorrichtung zur Ermittlung des zirkulierenden Blutvolumens nach dem Oberbegriff des Anspruchs 6.

Diesbezüglich liegt ihr die Aufgabe zugrunde, eine Vorrichtung zur Ermittlung des zirkulierenden Blutvolumens zu entwickeln, bei der der gesamte Informationsgehalt der optisch gemessenen Farbstoffverdünnungskurve für die Berechnung der Eliminationskinetik ausgenutzt wird.

Diese Aufgabe wird bei einer Vorrichtung nach dem Oberbegriff des Anspruchs 6 durch die im Kennzeichen angegebenen Merkmale gelöst.

Hinsichtlich der Wirkungsweise und der Vorteile der Ausgestaltungen der Vorrichtung gelten die Erläuterungen für das Verfahren entsprechend.

Weiterbildung und vorteilhafte Ausgestaltungen von Verfahren und Vorrichtung ergeben sich aus den Ansprüchen, der weiteren Beschreibung und der Zeichnung, anhand der die Erfindung näher beschrieben wird.

In der Zeichnung zeigen:

Fig. 1    eine graphische Darstellung eines Konzentrationszeitverlaufs eines Indikatorfarbstoffes,

Fig. 2    ein Blockschaltbild einer Vorrichtung zur Messung des zirkulierenden Blutvolumens nach der Erfindung,

Fig. 3    ein schematisches Modell zur Darstellung der Rezirkulation eines Indikators im Kreislauf,

Fig. 4    eine Darstellung der Kreislauftransportfunktion und

Fig. 5    eine Darstellung einer typischen Kreislaufimpulsantwort.

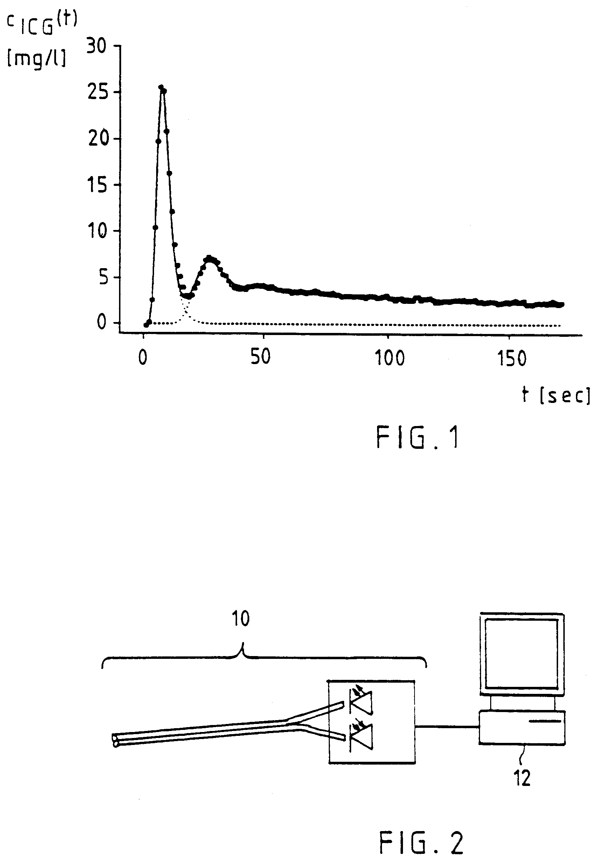

In Fig. 1 ist der Konzentrationszeitverlauf eines Indikatorfarbstoffs nach einer Injektion in den Blutkreislauf dargestellt. Es handelt sich um eine Indikatorverdünnungskurve bei Bolusinjektion von 0,2 mg/kg Indocyaningrün in den rechten Vorhof des Herzens und aortaler Messung.

Nach einer ersten Passage des Farbstoffes vom Injektionsort durch den Lungenkreislauf ist ein Wiederanstieg der Konzentration zu beobachten, der aus der Rezirkulation des Indikators resultiert. Erst nach mehreren Kreislaufpassagen des Farbstoffes kann die Verteilungsphase als abgeschlossen betrachtet werden. Nach den bisher bekannten Verfahren könnte erst danach aus dem langsamen, kaum sichtbaren Abfall der Indikatorkonzentration die Eliminationskinetik des Farbstoffes durch die Leber ermittelt werden.

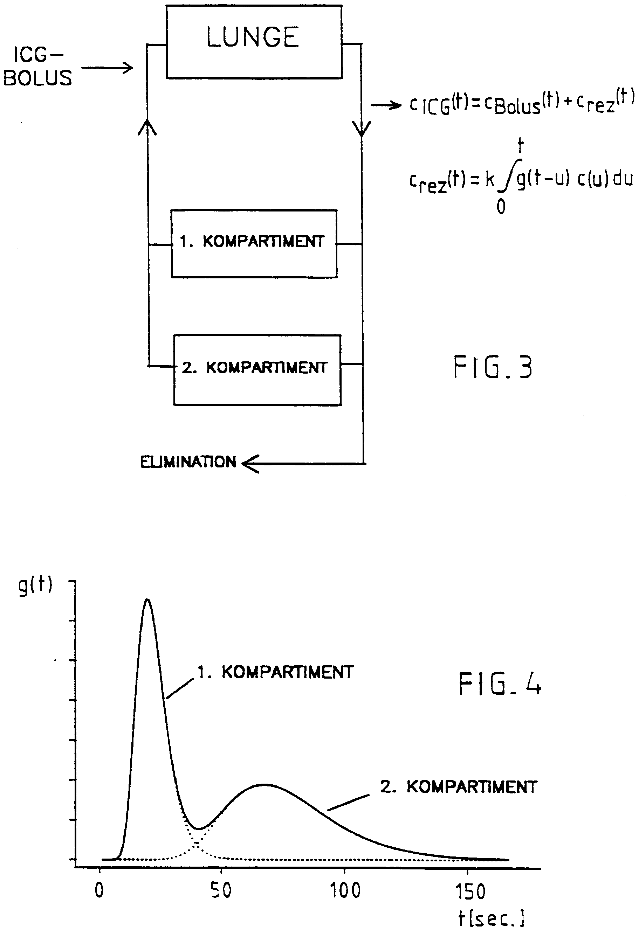

Demgegenüber wird bei der Erfindung der gesamte Informationsgehalt der Farbstoffverdünnungskurve ausgewertet. Hierzu dient die in Fig. 2 dargestellte Vorrichtung. Sie umfaßt einen fiberoptischen Meßsensor und einen damit verbundenen Rechner. Der fiberoptische Meßsensor wird in einem zentralen Blutgefäß angeordnet und nimmt unmittelbar nach Injizieren einer vorgegebenen Menge Indikatorfarbstoffes in den Blutkreislauf die Farbstoffkonzentrationskurve auf. In dem angeschlossenen Rechner wird aus dieser Kurve, die der in Fig. 1 gezeigten Darstellung entspricht, das zirkulierende Blutvolumen bestimmt, und zwar auf der Basis eines in Fig. 3 dargestellten Modells der Rezirkulation eines Indikators im Kreislauf.

Das Modell zeigt den Transportweg des injizierten Indikatorfarbstoffes ICG als Bolusinjektion der zuerst durch die Lunge führt. Hinter der Lunge teilt sich der Transportweg in ein erstes, schnelles Kompartiment, ein zweites langsames Kompartiment und in eine Elimination des Farbstoffes durch die Leber. Das erste und zweite Kompartiment führen anschließend wieder zur Lunge zurück. Die Konzentration des Indikatorfarbstoffes am Ausgang der Lunge folgt der Funktion:

$$c(t) = c_{bolus}(t) + c_{rez}(t)$$

mit

$$c_{rez}(t) = k * \int_{0}^{t} g(t-u) * c(u)\, du.$$

Hierbei bilden c(t) den Konzentrationszeitverlauf des Indikatorfarbstoffes, $c_{bolus}(t)$ der den Meßort direkt passierende erste Anteil des Farbstoffkonzentrationszeitverlaufs, $c_{rez}(t)$ einen rezirkulierender Anteil des Farbstoffkonzentrationszeitverlaufs und k die Eliminationsfraktion des durch die Leber eliminierten Farbstoffes. g(t) ist eine Transportfunktion, die das Transportverhalten des Kreislaufs charakterisiert.

Der Transportprozeß wird durch ein Faltungintegral beschrieben. Rezirkulation in dieser Darstellungsweise bedeutet, daß das Ergebnis der Faltung $c_{rez}(t)$ gleichzeitig auch die Eingangsfunktion c(t) beeinflußt. Die Rezirkulation des Indikators führt somit zu einem Zusammenhang, der im Prinzip folgendermaßen beschrieben ist:

$$c(t) = f[g(t), c(t)]$$

Für g(t) werden die Kompartimente des Kreislaufmodells angesetzt. Da Voruntersuchungen ergaben, daß bei Farbstoffverdünnungskurven, die an Patienten gemessen worden sind, in der Regel für g(t) zwei Kompartimente anzusetzen sind, ergibt sich für die allgemeine Modellfunktion

$$g(t) = a_m g_m(t) + a_{m+1} g_{m+1}(t) + \dots + a_n g_n(t)$$

$$\text{mit } \sum_{m=1}^{n} a_m = 1,$$

bei der die einzelnen Kompartimente $a_m g_m$ durch linksschiefe Verteilungsfunktionen beschrieben sind, der spezielle Ausdruck:

$$g(t) = a_1 g_1(t) + a_2 g_2(t).$$

Die Berechnung der Kreislauftransportfunktionen aus gemessenen Farbstoffkurven wird durch den Rechner mit Hilfe eines iterativen, nicht-linearen Anpassungsverfahrens durchgeführt werden, bei dem unter Vorgabe der Modellfunktion

$$g(t) = a_1 g_1(t) + a_2 g_2(t)$$

wiederholt eine rekursive Faltung gemäß den Gleichungen

$$c(t) = c_{bolus}(t) + c_{rez}(t)$$

und

$$c_{rez}(t) = k * \int_0^t g(t-u) * c(u) \, du$$

durchgeführt wird, wobei k, $a_1$, $a_2$ und die Parameter der Verteilungsfunktionen nach dem Verfahren der kleinsten quadratischen Abweichung optimiert werden.

Nach Durchführung dieser Rechenschritte ergibt sich eine Transportfunktion, wie sie als Beispiel für die in Fig. 1 enthaltenen Daten für zwei Kompartimente errechnet wurde und in Fig. 4 dargestellt ist.

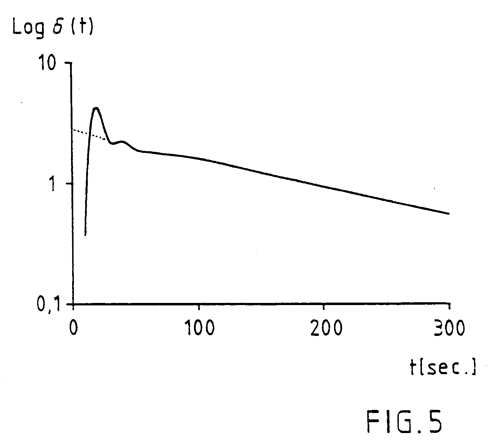

Für die ermittelte Kreislauftransportfunktion läßt sich nun die Kreislaufimpulsantwort berechnen, die einer idealen Indikatorinjektion in unendlich kurzer Zeit zum Zeitpunkt t = 0 entsprechen würde. Diese Kreislaufimpulsantwort ist in Fig. 5 dargestellt. Sie weist bei halblogarithmischer Auftragung nach einer initialen Verteilungsphase im weiteren Verlauf eine monoexponentielle Charakteristik auf, die eindeutig durch die Parameter der Kreislauftransportfunktion determiniert wird.

Es ist daher in Analogie zum konventionellen Verfahren möglich, die virtuelle Konzentration $c_0$ zum Zeitpunkt t = 0 durch monoexponentielle Rückextrapolation bzw. durch direkte Berechnung aus den Parametern der Kreislauftransportfunktion zu ermitteln.

Nunmehr ergibt sich das zirkulierende Blutvolumen $V_{d\text{-}Blut}$ aus der vorgegebenen Menge des injizierten Indikatorfarbstoffes $m_0$ und der virtuellen Farbstoffkonzentration $c_0$ zum Zeitpunkt t = 0 gemäß der Beziehung

$$V_{d\text{-}Blut} = m_0 / c_0.$$

Diese Berechnung erfolgt mit demselben Rechner, mit dem auch die Datenerfassung für die Farbstoffkonzentrationszeitkurven vorgenommen wird. Bei einem handelsüblichen MS-DOS Rechner dauert die Auswertung der Farbstoffkurven nur wenige Sekunden, sodaß das Ergebnis unmittelbar nach der Messung zur Verfügung steht.

## Patentansprüche

1. Verfahren zur Ermittlung des zirkulierenden Blutvolumens ($V_{d\text{-}Blut}$) nach Injektion einer vorgegebenen Menge ($m_0$) Indikatorfarbstoff in den Blutkreislauf, optische Messung der im Blutkreislauf resultierenden Farbstoffkonzentration ($c_0$) und Berechnung des Blutvolumens ($V_{d\text{-}Blut}$) entweder aus der vorgegebenen Menge ($c_0$) des injizierten Indikatorfarbstoffes und des Farbstoffkonzentrationszeitverlaufes oder aus dem Blutvolumenstrom und der aus dem Farbstoffkonzentrationszeitverlauf bestimmten mittleren Transitzeit ($m_{tt}$) des Indikatorfarbstoffes, **dadurch gekennzeichnet**, daß die Farbstoffkonzentration ($c_{(t)}$) bereits unmittelbar nach Injektion der vorgegebenen Menge ($m_0$) Indikatorfarbstoff in den Blutkreislauf gemessen wird und aus der Messung der Farbstoffkonzentration ($c_{(t)}$) eine Kreislauftransportfunktion ($g(t)$) berechnet wird, und daß über die Kreislauftransportfunktion ($g(t)$) entweder die Kreislaufimpulsantwort ($d(t)$) bestimmt wird, die einer idealen Indikatorinjektion in extrem kurzer Zeit zu einem Zeitpunkt (t = 0) entsprechen würde, und aus der Kreislaufimpulsantwort ($d(t)$) durch Rückextrapolation oder direkte Berechnung aus den Parametern der Kreislauftransportfunktion ($g(t)$) die virtuelle Farbstoffkonzentration ($c_0$) zum Zeitpunkt (t = 0) bestimmt wird und das Blutvolumen ($V_{d\text{-}Blut}$) aus der vorgegebenen Menge des injizierten Indikatorfarbstoffes ($m_0$) und der virtuellen Farbstoffkonzentration ($c_0$) zum Zeitpunkt (t = 0) gemäß der Beziehung

$$V_{d\text{-}Blut} = m_0 / c_0$$

berechnet wird oder daß die mittlere Transitzeit ($m_{tt}$) bestimmt wird, die der Indikatorfarbstoff benötigt, um den Kreislauf oder einen Teilkreislauf zu passieren und das Blutvolumen ($V_{d\text{-}Blut}$) aus dem Volumenstrom ($V_{Blut}$) und der mittleren Transitzeit ($m_{tt}$) gemäß der Beziehung

$$V_{d\text{-}Blut} = V_{Blut} * m_{tt}$$

bestimmt wird.

2. Verfahren nach Anspruch 1, **dadurch gekennzeichnet**, daß die Berechnung der Kreislauftransportfunktion ($g(t)$) aus der gemessenen Farbstoffkonzentration ($c_{(t)}$) mit Hilfe eines iterativen, nichtlinearen Anpassungsverfahrens durchgeführt wird, bei dem unter Vorgabe einer Modellfunktion

$$g(t) = a_m g_m(t) + a_{m+1} g_{m+1}(t) + \ldots + a_n g_n(t)$$

$$\text{mit} \sum_{m=1}^{n} a_m = 1,$$

bei der die einzelnen Kompartimente $a_m g_m$ durch linksschiefe Verteilungsfunktionen beschrieben sind, wiederholt eine rekursive Faltung gemäß den Gleichungen

$$c(t) = c_{bolus}(t) + c_{rez}(t)$$

und

$$c_{rez}(t) = k * \int_0^t g(t-u) * c(u) \, du$$

durchgeführt wird und die Parameter k, $a_m$ sowie die Parameter der Verteilungsfunktionen nach dem Verfahren der kleinsten quadratischen Abweichung optimiert werden, wobei wenigstens ein Kompartiment $a_1 g_1(t)$ angesetzt wird und c(t) den Konzentrationszeitverlauf des Indikatorfarbstoffes, $c_{bolus}(t)$ der den Meßort direkt passierende erste Anteil des Farbstoffkonzentrationszeitverlaufs, $c_{rez}(t)$ einen rezirkulierender Anteil des Farbstoffkonzentrationszeitverlaufs und k die Eliminationsfraktion des durch die Leber eliminierten Farbstoffes bilden.

3. Verfahren nach Anspruch 2, <u>dadurch gekennzeichnet</u>, daß zwei Kompartimente ($a_1 g_1(t)$) und ($a_2 g_2(t)$) angesetzt werden.

4. Verfahren nach einem oder mehreren der Ansprüche 1 bis 3, <u>dadurch gekennzeichnet</u>, daß die optische Messung der im Blutkreislauf resultierenden Farbstoffkonzentration durch fiberoptische Messung in einem zentralen Gefäß vorgenommen wird.

5. Verfahren nach einem oder mehreren der Ansprüche 1 bis 3, <u>dadurch gekennzeichnet</u>, daß die optische Messung der im Blutkreislauf resultierenden Farbstoffkonzentration nicht-invasiv mittels Licht-Transmissions- oder Reflexionsmessung eingestrahlten Lichtes an geeigneten Körperorten, insbesondere am Finger, Ohrläppchen, Nasenrücken, Wangenschleimhaut oder Stirn gemessen wird.

6. Vorrichtung zur Ermittlung des zirkulierenden Blutvolumens ($V_{d-Blut}$) mit einem optischen Meßsensor (10) und einem damit verbundenen Rechner (12), wobei mittels des Meßsensors (10) nach Injektion einer vorgegebenen Menge ($m_0$) Indikatorfarbstoffes in den Blutkreislauf die im Blutkreislauf resultierende Farbstoffkonzentration ($c_{(t)}$) gemessen wird und mittels des Rechners (12) das Blutvolumen ($V_{d-Blut}$) entweder aus der vorgegebenen Menge ($m_0$) des injizierten Indikatorfarbstoffes und des Farbstoffkonzentrationszeitverlaufes oder aus dem Blutvolumenstrom und der aus dem Farbstoffkonzentrationszeitverlauf bestimmten mittleren Transitzeit ($m_{tt}$) des Indikatorfarbstoffes bestimmbar ist, <u>dadurch gekennzeichnet</u>, daß der Rechner (12) so gesteuert ist, daß die Farbstoffkonzentration ($c_{(t)}$) bereits unmittelbar nach Injektion der vorgegebenen Menge ($m_0$) Indikatorfarbstoff in den Blutkreislauf gemessen wird und aus der Messung der Farbstoffkonzentration ($c_{(t)}$) eine Kreislauftransportfunktion (g(t)) berechnet wird, und daß über die Kreislauftransportfunktion (g(t)) entweder die Kreislaufimpulsantwort (d(t)) bestimmt wird, die einer idealen Indikatorinjektion in extrem kurzer Zeit zu einem Zeitpunkt (t = 0) entsprechen würde, und aus der Kreislaufimpulsantwort (d(t)) durch Rückextrapolation oder direkte Berechnung aus den Parametern der Kreislauftransportfunktion (g(t)) die virtuelle Farbstoffkonzentration ($c_0$) zum Zeitpunkt (t = 0) bestimmt wird und das Blutvolumen ($V_{d-Blut}$) aus der vorgegebenen Menge des injizierten Indikatorfarbstoffes ($m_0$) und der virtuellen Farbstoffkonzentration ($c_0$) zum Zeitpunkt (t = 0) gemäß der Beziehung

$$V_{d-Blut} = m_0 / c_0$$

berechnet wird oder daß die mittlere Transitzeit ($m_{tt}$) bestimmt wird, die der Indikatorfarbstoff benötigt, um den Kreislauf oder einen Teilkreislauf zu passieren und das Blutvolumen ($V_{d-Blut}$) aus dem Volumenstrom ($V_{Blut}$) und der mittleren Transitzeit ($m_{tt}$) gemäß der Beziehung

$$V_{d-Blut} = V_{Blut} * m_{tt}$$

bestimmt wird.

7. Vorrichtung nach Anspruch 6, <u>dadurch gekennzeichnet</u>, daß der Rechner (12) ferner so gesteuert ist, daß die Berechnung der Kreislauftransportfunktion (g(t)) aus der gemessenen Farbstoffkonzentration mit Hilfe eines iterativen, nichtlinearen Anpassungsverfahrens durchgeführt wird, bei dem unter Vorgabe einer Modellfunktion

$$g(t) = a_m g_m(t) + a_{m+1} g_{m+1}(t) + \dots + a_n g_n(t)$$

$$\text{mit } \sum_{m=1}^{n} a_m = 1,$$

bei der die einzelnen Kompartimente $a_m g_m$ durch linksschiefe Verteilungsfunktionen beschrieben sind, wiederholt eine rekursive Faltung gemäß den Gleichungen

$$c(t) = c_{bolus}(t) + c_{rez}(t)$$

und

$$c_{rez}(t) = k * \int_0^t g(t-u) * c(u)\, du$$

durchgeführt wird und die Parameter k, $a_m$ sowie die Parameter der Verteilungsfunktionen nach dem Verfahren der kleinsten quadratischen Abweichung optimiert werden, wobei wenigstens ein Kompartiment $a_1 g_1(t)$ angesetzt wird und c(t) den Konzentrationszeitverlauf des Indikatorfarbstoffes, $c_{bolus}(t)$ der den Meßort direkt passierende erste Anteil des Farbstoffkonzentrationszeitverlaufs, $c_{rez}(t)$ einen rezirkulierender Anteil des Farbstoffkonzentrationszeitverlaufs und k die Eliminationsfraktion des durch die Leber eliminierten Farbstoffes bilden.

8. Vorrichtung nach Anspruch 7, <u>dadurch gekennzeichnet</u>, daß der Rechner (12) so gesteuert ist, daß zwei Kompartimente ($a_1 g_1(t)$) und ($a_2 g_2(t)$) angesetzt werden.

9. Vorrichtung nach einem oder mehreren der Ansprüche 6 bis 8, <u>dadurch gekennzeichnet</u>, daß der optische Meßsensor (10) ein fiberoptischer Katheter ist, der zur Messung in einem zentralen Gefäß angeordnet wird.

10. Vorrichtung nach einem oder mehreren der Ansprüche 6 bis 8, <u>dadurch gekennzeichnet</u>, daß der optische Meßsensor (10) einen Lichtsender und Lichtempfänger zur nichtinvasiven Licht-Transmissions- oder Reflexionsmessung eingestrahlten Lichtes an geeigneten Körperorten, insbesondere am Finger, Ohrläppchen, Nasenrücken, Wangenschleimhaut oder Stirn umfaßt.

## Claims

1. Method of determining the volume of blood in circulation ($V_{d-Blut}$) after injection of a predetermined quantity ($m_O$) of indicator dye into the blood stream, optical measurement of the resultant dye concentration ($c_O$) in the blood stream and calculation of the blood volume ($V_{d-Blut}$) either from the predetermined quantity ($c_O$) of the injected indicator dye and from the dye concentration time pattern or from the volumetric flow rate of the blood and the mean transit time ($m_{tt}$) of the indicator dye determined from the dye concentration time pattern, characterized in that the dye concentration (c(t)) is measured immediately after injection of the predetermined quantity ($m_o$) of indicator dye into the blood stream and from the measurement of the dye concentration (c(t)) a blood stream transport function (g(t)) is calculated, and that from the blood stream transport function (g(t)) either the circulatory pulse response (d(t)), which would correspond to an ideal indicator injection in an extremely short time at an instant (t = O), is determined, and from the circulatory pulse response (d(t)), the virtual dye concentration ($c_O$) at the instant (t = 0) is determined by back-extrapolation or direct computation from the parameters of the blood stream transport function (g(t)), and the blood volume ($V_{d-Blut}$) is calculated from the predetermined quantity of the injected indicator dye ($m_O$) and the virtual dye concentration ($c_O$) at the instant (t = 0) in accordance with the relationship

$$V_{d-Blut} = m_O / c_O$$

or that the mean transit time ($m_{tt}$) is determined, that is required by the indicator dye for passing around the circuit or a part of the circuit and the blood volume ($V_{d-Blut}$) is determined from the volumetric flow rate ($V_{Blut}$) and the mean transit time ($m_{tt}$) in accordance with the relationship

$$V_{d\text{-}Blut} = V_{Blut} \cdot m_{tt}.$$

2. Method according to claim 1, characterized in that the computation of the blood stream transport function (g(t)) is carried out from the measured dye concentration (c(t)) by means of an iterative, non-linear adaptation process, in which, with a given model function

$$g(t) = a_m g_m(t) + a_{m+1} g_{m+1}(t) + \dots + a_n g_n(t)$$

$$\text{where} \sum_{m=1}^{n} a_m = 1$$

in which the individual compartments $a_m g_m$ are described by left-inclined distribution functions, a repetitive convolution is repeatedly carried out in accordance with the equations

$$c(t) = c_{bolus}(t) + c_{rez}(t)$$

and

$$c_{rez}(t) = k \cdot \int_0^t g(t\text{-}u) \cdot c(u)\, du$$

and the parameters k, $a_m$ and also the parameters of the distribution functions are optimized by the method of the smallest square deviation, wherein at least one compartment $a_1 g_1(t)$ is used and c(t) respresents the concentration time pattern of the indicator dye, $c_{bolus}(t)$ represents the first portion of the dye concentration time pattern passing directly through the measurement position, $c_{rez}(t)$ represents a recirculating portion of the dye concentration time pattern and k represents the elimination fraction of the dye eliminated by the liver.

3. Method according to claim 2, characterized in that two compartments ($a_1 g_1(t)$) and $a_2 g_2(t)$) are used.

4. Method according to one or more of claims 1 to 3, characterized in that the optical measurement of the resultant dye concentration in the blood stream is performed by fibre-optic measurement in a central vessel.

5. Method according to one or more of claims 1 to 3, characterized in that the optical measurement of the resultant dye concentration in the blood stream is measured non-invasively by means of light transmission or reflection measurement of incident light at suitable positions of the body, especially at the finger, ear lobe, bridge of the nose, mucus membrane of the cheek or forehead.

6. Device for determining the volume of blood in circulation ($V_{d\text{-}Blut}$), comprising an optical measuring sensor (10) and a computer (12) connected to it, wherein by means of the measuring sensor (10), after injection of a predetermined quantity ($m_O$) of indicator dye into the blood stream, the resultant dye concentration (c(t)) in the blood stream is measured and, by means of the computer (12), the blood volume ($V_{d\text{-}blut}$) can be determined either from the predetermined quantity ($m_O$) of the injected indicator dye and the dye concentration time pattern or from the blood volumetric flow rate and the mean transit time ($m_{tt}$) of the indicator dye determined from the dye concentration time pattern, characterized in that the computer (12) is so controlled that the dye concentration (c(t)) is measured immediately after injection of the predetermined quantity ($m_O$) of indicator dye into the blood stream and, from the measurement of the dye concentration (c(t)), a blood stream transport function (g(t)) is calculated, and that by the use of the blood stream transport function (g(t)), either the circulatory pulse response (d(t)), which would correspond to an ideal indicator injection in an extremely short time at an instant (t = 0), is determined, and from the circulatory pulse response (d(t)) the virtual dye concentration ($c_O$) at the instant (t = 0) is determined by back-extrapolation or direct computation from the parameters of the blood stream transport function (g(t)), and the blood volume ($V_{d\text{-}Blut}$) is calculated from the predetermined quantity of the injected indicator dye ($m_O$) and the virtual dye concentration ($c_O$) at the instant (t = 0) according to the relationship

$$V_{d\text{-}Blut} = m_O / c_O$$

or the mean transit time ($m_{tt}$), which the indicator dye requires for passing around the circuit or a part of the circuit, is determined, and the volume of blood ($V_{d\text{-}Blut}$) is determined from the volumetric flow rate ($V_{Blut}$) and the mean transit time ($m_{tt}$) according to the relationship

$$V_{d\text{-}Blut} = V_{Blut} \cdot m_{tt}.$$

**7.** Device according to claim 6, characterized in that the computer (12) is furthermore so controlled that the computation of the blood stream transport function (g(t)) is carried out from the measured dye concentration by means of an iterative, non-linear adaptation process, in which, with a given model function

$$g(t) = a_m g_m(t) + a_{m+1} g_{m+1}(t) + \dots + a_n g_n(t)$$

$$\text{where } \sum_{m=1}^{n} a_m = 1,$$

in which the individual compartments $a_m g_m$ are described by left-inclined distribution functions, a repetitive convolution is repeatedly carried out according to the equations

$$c(t) = c_{bolus}(t) + c_{rez}(t)$$

and

$$c_{rez}(t) = k \cdot \int_0^t g(t-u) \cdot c(u) \, du$$

and the parameters k, $a_m$ and also the parameters of the distribution functions are optimized by the method of the smallest square deviation, wherein at least one compartment $a_1 g_1(t)$ is used and c(t) represents the concentration time pattern of the indicator dye, $c_{bolus}(t)$ represents the first portion of the dye concentration time pattern directly passing through the measurement position, $c_{rez}(t)$ represents a recirculating portion of the dye concentration time pattern and k represents the elimination fraction of the dye eliminated by the liver.

**8.** Device according to claim 7, characterized in that the computer (12) is so controlled that two compartments $(a_1 g_1(t))$ and $(a_2 g_2(t))$ are used.

**9.** Device according to one or more of claims 6 to 8, characterized in that the optical measuring sensor (10) is a fibre-optic catheter, which is arranged for measurement on a central vessel.

**10.** Device according to one or more of claims 6 to 8, characterized in that the optical measuring sensor (10) comprises a light transmitter and light receiver for non-invasive light transmission or reflection measurement of incident light at suitable parts of the body, especially at the finger, ear lobe, bridge of the nose, mucus membrane of the cheek or forehead.

**Revendications**

**1.** Procédé pour déterminer le volume de sang en circulation $(V_{d\text{-}sang})$, après injection d'une quantité prédéterminée $(m_0)$ d'un colorant indicateur dans la circulation sanguine, par la mesure optique de la concentration de colorant $(c_0)$ gui en résulte dans la circulation sanguine et le calcul du volume de sang $(V_{d\text{-}sang})$, soit à partir de la quantité prédéterminée $(c_0)$ du colorant indicateur et du cycle de concentration du colorant, soit à partir du courant de volume de sang et du temps de transit moyen $(m_{tt})$ du colorant indicateur déterminé à partir du cycle de concentration du colorant, caractérisé en ce que la concentration du colorant $(c_{(t)})$ est immédiatement mesurée après injection de la quantité prédéterminée $(m_0)$ de colorant indicateur dans la circulation sanguine et une fonction de transport circulatoire (g(t)) est calculée à partir de la mesure de la concentration du colorant $(c_{(t)})$, et en ce que, par la fonction de transport circulatoire (g(t)), est déterminée la réponse impulsionnelle circulatoire (d(t)) qui correspondrait à une injection d'indicateur idéale en un temps extrêmement court à un moment (t = 0), et qu'à partir de la réponse impulsionnelle (d(t)) par extrapolation inverse ou par calcul direct à partir des paramètres de la fonction de transport circulatoire (g(t)), est définie la concentration du colorant virtuelle $(c_0)$ au moment (t = 0) et que le volume de sang $(V_{d\text{-}sang})$ est calculé à partir de la quantité prédéterminée de colorant indicateur injecté $(m_0)$ et de la concentration du colorant virtuelle $(c_0)$ au moment (t = 0) suivant la relation:

$$V_{d\text{-}sang} = m_0 / c_0$$

ou en ce qu'est défini le temps de transit moyen ($m_{tt}$) qui est nécessaire au colorant indicateur pour parcourir le cycle de circulation ou une partie du cycle de circulation et que le volume de sang ($V_{d\text{-}sang}$) est déterminé à partir du débit volumique ($V_{sang}$) et du temps de transit moyen ($m_{tt}$) par la relation:

$$V_{d\text{-}sang} = V_{sang} * m_{tt}$$

**2.** Procédé suivant la revendication 1, <u>caractérisé en ce</u> que le calcul de la fonction de transport circulatoire ($g_{(t)}$) est réalisé à partir de la concentration de colorant mesurée ($c_{(t)}$) au moyen d'un procédé de correction itératif, non linéaire dans lequel sous donnée préalable d'une fonction modèle:

$$g(t) = a_m g_m(t) + a_{m+1} g_{m+1}(t) + ... + a_n g_n(t)$$

$$\text{avec} \sum_{m=1}^{n} a_m = 1,$$

dans laquelle les coefficients individuels $a_n g_n$ sont décrits par des fonctions de répartition inclinéen à gauche, avec répétition est réalisée une convolution récursive suivant l'équation:

$$c(t) = c_{bolus}(t) + c_{rez}(t)$$

et

$$c_{rez}(t) = k + \int_0^t g(t\text{-}u) * c(u) \, du$$

et les paramètres k, $a_m$ ainsi que les paramètres des fonctions de répartition sont optimisés d'après le procédé de la plus petite déviation quadratique, au moins un coefficient $a_1 g_1(t)$ étant ajouté, et c(t) formant le cycle de concentration du colorant indicateur, $c_{bolus}(t)$ la première partie du cycle de concentration du colorant passant directement au point de mesure, $c_{rez}(t)$ une partie de recirculation du cycle de concentration du colorant et k la fraction d'élimination du colorant éliminé par le foie.

**3.** Procédé suivant la revendication 2, <u>caractérisé en ce</u> que deux coefficients ($a_1 g_1(t)$) et ($a_2 g_2(t)$) sont ajoutés.

**4.** Procédé suivant l'une ou plusieurs des revendications 1 à 3, <u>caractérisé en ce</u> que la mesure optique de la concentration du colorant qui en résulte dans la circulation sanguine est effectuée par mesure sur fibre optique dans un récipient central.

**5.** Procédé suivant l'une ou plusieurs des revendications 1 à 3, <u>caractérisé en ce</u> que la mesure optique de la concentration du colorant qui en résulte dans la circulation sanguine est mesurée sans pénétration au moyen de mesure par transmission ou réflexion de lumière incidente sur des endroits du corps, en particulier sur le doigt, le lobe de l'oreille, le dos du nez, la muqueuse malaire ou le front.

**6.** Procédé pour déterminer le volume de sang en circulation ($V_{d\text{-}sang}$) avec un capteur optique de mesure (10) et un calculateur (12) qui lui est relié, au moyen du capteur de mesure (10) après injection d'une quantité prédéterminée ($m_0$) d'un colorant indicateur dans la circulation sanguine, par la concentration de colorant ($c_{(t)}$) qui en résulte dans la circulation sanguine étant mesurée et, au moyen du calculateur (12), le volume de sang ($V_{d\text{-}sang}$) pouvant être déterminé soit à partir de la quantité prédéterminée ($C_0$) du colorant indicateur injecté et du cycle de concentration du colorant, soit à partir du courant de volume de sang et du temps de transit moyen ($m_{tt}$) du colorant indicateur déterminé à partir du cycle de concentration du colorant, <u>caractérisé en ce</u> que le calculateur (12) est commandé de manière que la concentration du colorant ($C_{(t)}$ soit immédiatement mesurée après injection de la quantité prédéterminée ($m_0$) de colorant indicateur dans la circulation sanguine et qu'une fonction de transport circulatoire (g(t)) est calculée à partir de la mesure de la concentration du colorant ($C_{(t)}$), et en ce que, par la fonction de transport circulatoire (g(t)), est déterminée la réponse impulsionnelle circulatoire (d(t)) qui correspondrait à une injection d'indicateur idéale en un temps extrêmement court à un moment (t = 0), et qu'à partir de la réponse impulsionnelle (d(t)) par extrapolation inverse ou par calcul direct à partir des paramètres de la fonction de transport circulatoire (g(t)), est définie la concentration du colorant virtuelle ($c_0$) au moment (t = 0) et que le volume de sang ($V_{d\text{-}sang}$) est calculé à partir de la quantité prédéterminée de colorant indicateur injecté ($m_0$) et de la concentration du colorant virtuelle ($c_0$) au moment (t = 0) suivant la relation:

$$V_{d\text{-}sang} = m_0 / c_0$$

ou en ce que le temps de transit moyen ($m_{tt}$) est défini qui est nécessaire au colorant indicateur pour parcourir le cycle de circulation ou une partie du cycle de circulation et que le volume de sang ($V_{d\text{-}sang}$) est déterminé à partir du débit volumique ($V_{sang}$) et du temps de transit moyen ($m_{tt}$) par la relation:

$$V_{d\text{-}sang} = V_{sang} * m_{tt}$$

7. Procédé suivant la revendication 6, <u>caractérisé en ce</u> que le calculateur (12) est en outre commandé de manière que le calcul de la fonction de transport circulatoire ($g_{(t)}$) est réalisé à partir de la concentration de colorant mesurée ($c_{(t)}$) au moyen d'un procédé de correction itératif, non linéaire dans lequel sous donnée préalable d'une fonction modèle:

$$g(t) = a_m g_m(t) + a_{m+1} g_{m+1}(t) + ... + a_n g_n(t)$$

$$\text{avec} \sum_{m=1}^{n} a_m = 1,$$

dans laquelle les coefficients individuels $a_n g_n$ sont décrits par des fonctions de répartition inclinées à gauche, avec répétition est réalisée une convolution récursive suivant l'équation:

$$c(t) = c_{bolus}(t) + c_{rez}(t)$$

et

$$c_{rez}(t) = k + \int_0^t g(t\text{-}u) * c(u) \, du$$

et les paramètres k, $a_m$ ainsi que les paramètres des fonctions de répartition sont optimisés d'après le procédé de la plus petite déviation quadratique, au moins un coefficient $a_1 g_1(t)$ étant ajouté, et c(t) formant le cycle de concentration du colorant indicateur, $c_{bolus}(t)$ la première partie du cycle de concentration du colorant passant directement au point de mesure, $c_{rez}(t)$ une partie recirculante du cycle de concentration du colorant et k la fraction d'élimination du colorant éliminé par le foie.

8. Procédé suivant la revendication 7, <u>caractérisé en ce</u> que le calculateur (12) est commandé de manière que deux coefficients ($a_1 g_1(t)$) et ($a_2 g_2(t)$) soient ajoutés.

9. Procédé suivant l'une ou plusieurs des revendications 6 à 8, <u>caractérisé en ce</u> que le capteur optique de mesure (10) est un cathéter en fibre optique qui est disposé pour faire la mesure dans un récipient central.

10. Procédé suivant l'une ou plusieurs des revendications 6 à 8, <u>caractérisé en ce</u> que le capteur optique de mesure (10) comprend un émetteur de lumière et un récepteur de lumière sans invasion au moyen de mesure par transmission ou réflexion de lumière incidente sur des endroits du corps, en particulier sur le doigt, le lobe de l'oreille, le dos du nez, la muqueuse malaire ou le front.

FIG. 1

FIG. 2

ICG—
BOLUS $\rightarrow$

LUNGE

$\rightarrow c_{ICG}(t) = c_{Bolus}(t) + c_{rez}(t)$

$$c_{rez}(t) = k \int_0^t g(t-u)\, c(u)\, du$$

1. KOMPARTIMENT

2. KOMPARTIMENT

FIG.3

ELIMINATION $\leftarrow$

$g(t)$

1. KOMPARTIMENT

FIG.4

2. KOMPARTIMENT

0    50    100    150

$t$[sec.]

FIG. 5